(19) Europäisches Patentamt

European Patent Office

Office européen des brevets

(11) **EP 0 539 258 B1**

(12) **FASCICULE DE BREVET EUROPEEN**

(45) Date de publication et mention
de la délivrance du brevet:
**04.02.1998 Bulletin 1998/06**

(51) Int. Cl.⁶: **A61N 1/365**

(21) Numéro de dépôt: **92402760.0**

(22) Date de dépôt: **09.10.1992**

(54) **Procédé de réglage automatique de l'hystérésis dans un stimulateur cardiaque**

Verfahren zur automatischen Regelung einer Herzschrittmacherhysterese

Way of hysteresis automatic adjustment in a pacemaker

(84) Etats contractants désignés:
**BE CH DE ES GB IT LI SE**

(30) Priorité: **25.10.1991 FR 9113177**

(43) Date de publication de la demande:
**28.04.1993 Bulletin 1993/17**

(73) Titulaire:
**ELA MEDICAL (Société anonyme)
F-92541 Montrouge Cédex (FR)**

(72) Inventeurs:
• **Limousin, Marcel
F-92120 Montrouge (FR)**
• **Bouhour, Anne
F-75013 Paris (FR)**

(74) Mandataire: **Laget, Jean-Loup
Cabinet Loyer,
78, avenue Raymond Poincaré
75116 Paris (FR)**

(56) Documents cités:
EP-A- 0 326 629          EP-A- 0 398 488
EP-A- 0 422 271          US-A- 3 478 746
US-A- 3 656 487          US-A- 3 661 157
US-A- 3 678 937          US-A- 3 693 626
US-A- 4 030 510

Remarques:
Le dossier contient des informations techniques présentées postérieurement au dépôt de la demande et ne figurant pas dans le présent fascicule.

EP 0 539 258 B1

## Description

L'invention a pour objet un procédé de réglage automatique de l'hystérésis dans un stimulateur cardiaque, notamment en fonction de la fréquence du rythme auriculaire et de sa nature, stimulée ou spontanée.

Dans un stimulateur cardiaque, on définit la fréquence d'échappement comme la fréquence du stimulateur en l'absence d'activité cardiaque détectée. A cette fréquence d'échappement exprimée en cpm (coups par minute) correspond un intervalle d'échappement, exprimé en ms (millisecondes).

Si l'on prend comme exemple le cas de l'oreillette, le rythme auriculaire ne peut être sinusal qu'au dessus de la fréquence d'échappement. Si le rythme sinusal devient inférieur à la fréquence d'échappement, l'oreillette est stimulée.

Le but de l'hystérésis est d'autoriser le rythme sinusal à descendre dans certaines limites au dessous de la fréquence d'échappement sans pour autant stimuler. Autrement dit, l'hystérésis allonge l'intervalle d'échappement d'une durée supplémentaire, pour favoriser l'expression du rythme cardiaque spontané. L'hystérésis s'exprime en ms ou en pourcentage de la fréquence d'échappement.

Après une activité détectée, le stimulateur fonctionne comme si la fréquence d'échappement était réduite du pourcentage programmé de l'hystérésis. A titre d'exemple, dans le cas d'une fréquence d'échappement de 75 cpm, une hystérésis programmée à 10% abaisse la limite inférieure pour un rythme naturel à :

$$75 \times 90\% = 67,5 \text{ cpm.}$$

Dans les stimulateurs cardiaques mettant en oeuvre une hystérésis, comme celui décrit dans le document GB 1 351 995, dès que le coeur doit être stimulé, on revient à la fréquence d'échappement initiale jusqu'à l'apparition d'une nouvelle détection.

Dans ces stimulateurs, l'hystérésis est programmable, mais elle n'est pas réglable.

Le document EP 0 326 629 divulgue un stimulateur cardiaque muni d'une fonction d'hystérésis, dont l'intervalle d'échappement est variable (cas du stimulateur asservi), et où l'allongement de l'intervalle d'échappement est une fonction de l'intervalle d'échappement instantané.

Usuellement, l'hystérésis est programmée lors de l'initialisation du stimulateur et n'est pas modifiée en cours de fonctionnement. Il peut ainsi arriver que la fréquence d'échappement corrigée par l'hystérésis soit très proche du rythme spontané et que les stimulations observées résultent d'un conflit entre rythme spontané et fréquence d'échappement, et non d'une nécessité réelle de stimuler. Inversement, on peut observer des détections à une fréquence sensiblement éloignée de la fréquence d'échappement diminuée de l'hystérésis. Dans le premier cas, le taux d'hystérésis pourrait avantageusement être augmenté pour favoriser le rythme spontané, dans le second cas, il pourrait être diminué.

Le but de l'invention est de proposer un procédé de réglage automatique de l'hystérésis au cours du fonctionnement du stimulateur.

L'invention a pour objet un procédé de réglage automatique de l'hystérésis (H), autrement dit de la durée supplémentaire allongeant l'intervalle d'échappement (IE), dans un stimulateur cardiaque comportant un compteur de stimulations (M), procédant par décrément à partir d'un maximum programmé, et un compteur de détections (N), procédant par décrément à partir d'un maximum programmé, lesdits compteurs étant contrôlés par un logiciel, ladite durée supplémentaire (H) étant susceptible d'être réglée entre une valeur minimale et une valeur maximale, avec une marge (Ma) programmée définie comme un pourcentage de l'intervalle d'échappement, caractérisé en ce que

- le compteur de stimulations (M) est décrémenté après une stimulation et il est remis à son maximum après une détection,

- le compteur de détections (N) est décrémenté après une détection si l'intervalle entre deux détections successives est inférieur à l'intervalle d'échappement augmenté de l'hystérésis et diminué de la marge programmée ( IE+H-Ma ), et il est remis à son maximum après une stimulation, et

- le réglage de ladite durée supplémentaire (H) s'effectue suivant un des deux groupes de conditions suivantes :
  si

  . après une détection,
  . la durée supplémentaire (H) est inférieure à la valeur maximale diminuée d'un incrément (max),
  . et le compteur de stimulations indique une valeur (M) comprise entre 0 et le maximum programmé, limites exclues, alors on augmente d'un incrément ladite durée supplémentaire (H),
  et si
  . après une détection,
  . la durée supplémentaire (H) est supérieure à la valeur minimale augmentée d'un décrément (min),
  . et le compteur de détections (N) est à 0,

  alors on diminue d'un décrément ladite durée supplémentaire (H).

Selon l'invention :

- l'incrément, ou le décrément, est de préférence compris entre 10 et 50 ms ;

- lorsque, après une détection, l'intervalle (PP) entre deux détections successives est compris entre d'une part l'intervalle d'échappement augmenté de l'hystérésis et diminué de la marge programmée ( IE + H - Ma ), et d'autre part l'intervalle d'échappement augmenté de l'hystérésis (IE + H), alors le compteur de détection est remis à son maximum programmé.

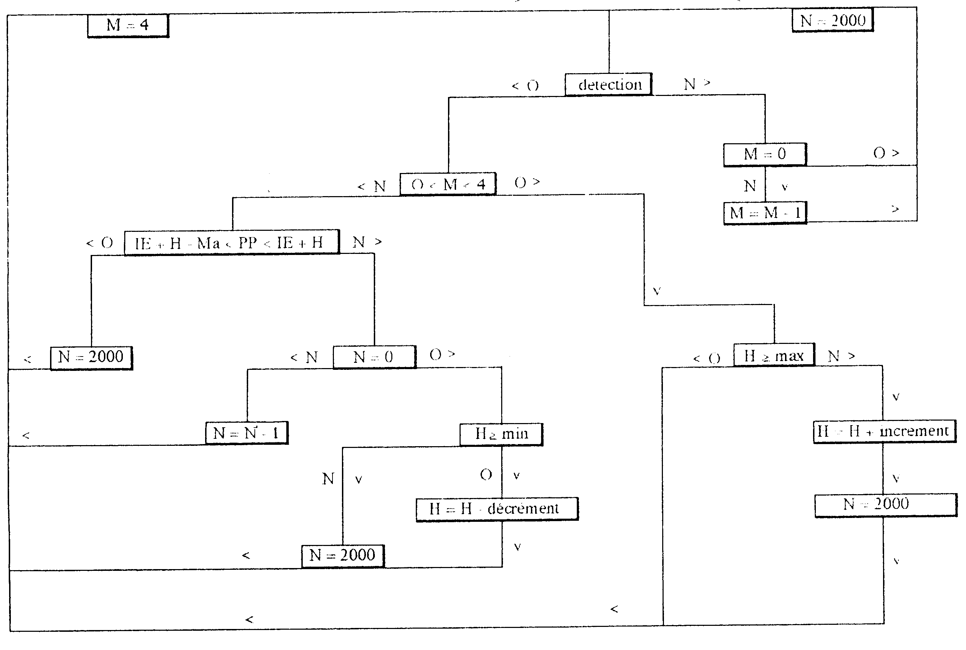

Pour résumer les différentes étapes du procédé de réglage automatique de l'hystérésis dans un stimulateur cardiaque selon l'invention, on a représenté au dessin annexe un schéma logique auquel il est fait référence dans la suite de la description.

Dans le schéma logique les abréviations ont la signification suivante :

H    : Hystérésis.
Ma   : Marge.
IE    : Intervalle d'échappement.
PP   : Intervalle entre deux ondes P successives.
M    : Indication du compteur de stimulations.
N    : Indication du compteur de détections.
max   : Hystérésis maximale diminuée d'un incrément.
min   : Hystérésis minimale augmentée d'un décrément.

Pour la mise en oeuvre du procédé selon l'invention, le stimulateur cardiaque est muni d'un compteur M de stimulations, auriculaires par exemple, qui procède par décrément à partir d'un maximum programmé, compris entre 1 et 10 et de préférence égal à 4. Il est également pourvu d'un compteur N de détections, auriculaires par exemple, qui procède par décrément à partir d'un maximum programmé, égal par exemple à 2000. Dans ce stimulateur, l'hystérésis est réglable entre une valeur minimale et une valeur maximale , ces deux valeurs pouvant être approchées avec une marge programmée.

Le réglage automatique de l'hystérésis est assuré par un logiciel qui assure l'exécution des fonctions suivantes :

A - Après une stimulation, le compteur N de détections est remis à son maximum programmé, par exemple 2000 ;
B - Après une stimulation, si le compteur M de stimulations n'est pas à 0, il est décrémenté de 1 ;
C - Après une détection, le compteur M de stimulations est remis à son maximum programmé, par exemple 4 ;
D - Après une détection, si le compteur M de stimulations n'est pas à 0, et si l'intervalle PP entre deux ondes P successives est compris entre d'une part l'intervalle d'échappement augmenté de l'hystérésis et diminué de la marge programmée ( IE + H - Ma ), et d'autre part l'intervalle d'échappement augmenté de l'hystérésis (IE + H), alors le compteur N de détection est remis à son maximum programmé, par exemple 2000.
E - Après une détection, si le compteur N de détections n'est pas à 0, et si l'intervalle entre deux ondes P successives est inférieur à l'intervalle d'échappement augmenté de l'hystérésis et diminué de la marge programmée ( IE + H - Ma ), alors le compteur N de détections est décrémenté de 1.
F - Après une détection, lorsque l'hystérésis est inférieure à l'hystérésis maximale diminuée d'un incrément, et lorsque, simultanément, le compteur M de stimulations est compris entre 0 et son maximum, alors l'hystérésis est augmentée d'un incrément, et le compteur M de stimulations est mis à son maximum, de préférence égal à 4.
G - Lorsque l'hystérésis est supérieure à l'hystérésis minimale augmentée d'un décrément et lorsque, simultanément, le compteur N de détections est à 0, alors l'hystérésis est diminuée d'un décrément, et le compteur N de détections est mis au maximum.

Avec le procédé de réglage selon l'invention, l'hystérésis est augmentée chaque fois qu'on détecte un petit nombre de stimulations consécutives, avant de récupérer une détection ; cela peut être le signe que la fréquence d'échappement est trop proche du rythme spontané: l'hystérésis est augmentée pour favoriser le rythme spontané.

Par ailleurs, l'hystérésis est diminuée quand on observe un grand nombre de détections consécutives, à une fréquence relativement éloignée de la fréquence d'échappement diminuée de l'hystérésis.

A titre d'exemple, l'hystérésis peut aller de 0 à 15% de la fréquence d'échappement avec une marge de 2%.

**Revendications**

1. Procédé de réglage automatique de l'hystérésis (H), autrement dit de la durée supplémentaire allongeant l'intervalle d'échappement (IE), dans un stimulateur cardiaque comportant un compteur de stimulations (M), procédant par décrément à partir d'un maximum programmé, et un compteur de détections (N), procédant par décrément à partir d'un maximum programmé, lesdits compteurs étant contrôlés par un logiciel, ladite durée supplémentaire (H) étant susceptible d'être réglée entre une valeur minimale et une valeur maximale, avec une marge (Ma) programmée définie comme un pourcentage de l'intervalle d'échappement, caractérisé en ce que

- le compteur de stimulations (M) est décrémenté après une stimulation et il est remis à son maximum après une détection,

- le compteur de détections (N) est décrémenté après une détection si l'intervalle (PP) entre deux détections successives est inférieur à l'intervalle d'échappement augmenté de l'hystérésis et diminué de la marge programmée ( IE+H-Ma ), et il est remis à son maximum après une stimulation, et

- le réglage de ladite durée supplémentaire (H)s'effectue suivant un des deux groupes de conditions suivantes :
  si

  . après une détection,

  . la durée supplémentaire (H) est inférieure à la valeur maximale diminuée d'un incrément (max),

  . et le compteur de stimulations indique une valeur (M) comprise entre 0 et le maximum programmé, limites exclues, alors on augmente d'un incrément ladite durée supplémentaire (H),
  et si

  . après une détection,

  . la durée supplémentaire (H) est supérieure à la valeur minimale augmentée d'un décrément (min),

  . et le compteur de détections (N) est à 0,

  alors on diminue d'un décrément ladite durée supplémentaire (H).

2. Procédé selon la revendication 1, caractérisé en ce que l'incrément, ou le décrément, est de préférence compris entre 10 et 50 ms.

3. Procédé selon la revendication 1, caractérisé en ce que, lorsque, après une détection, l'intervalle (PP) entre deux détections successives est compris entre d'une part l'intervalle d'échappement augmenté de l'hystérésis et diminué de la marge programmée ( IE + H - Ma ), et d'autre part l'intervalle d'échappement augmenté de l'hystérésis (IE + H), alors le compteur de détection est remis à son maximum programmé.

**Claims**

1. Method for the automatic regulation of the hysteresis, in other words of the additional duration extending the escape interval (IE), in a pacemaker comprising a stimulus counter (M) proceeding by decrements from a programmed maximum, and a detection counter (N) proceeding by decrements from a programmed maximum, said counters being controlled by a software programme, said additional duration (H) being susceptible of being set between a minimum value and a maximum value, with a programmed margin (Ma) defined as a percentage of the escape interval, characterized in that:

- the stimulus counter (M) is decremented subsequent to a stimulus and is reset to its maximum subsequent to a detection,
- the detection counter (N) is decremented subsequent to a detection if the interval (PP) between two successive detections is shorter than the escape interval increased by the hysteresis and decreased by the programmed margin ( IE+H-Ma ), and is reset to its maximum subsequent to a stimulus, and
- the setting of said additional duration (H) is performed in accordance with one of the following two sets of conditions;
  if,

  • subsequent to a detection,
  • the additional duration (H) is lower than the maximum value less one increment (max.),
  • and the stimulus counter indicates a value (M) included between 0 and the programmed maximum, exclusive of the limits, then said additional duration (H) is increased by one increment,
  and if,
  • subsequent to a detection,
  • the additional duration (H) is greater than the minimum value increased by one increment (min.),
  • and the detections counter (N) is at 0,

  then said additional duration (H) is decreased by one decrement.

2. Method as claimed in claim 1, characterized in that the increment, or decrement, is preferably included between 10 and 50 ms.

3. Method as claimed in claim 1, characterized in that, when, subsequent to a detection, the interval (PP) between two successive detections is included between, on the one hand, the escape interval increased by the hysteresis and decreased by the programmed margin ( IE + H - Ma ), and, on the other hand, the escape interval increased by the hysteresis (IE + H), then the detection counter is reset to its programmed maximum.

**Patentansprüche**

1. Verfahren zur automatischen Regelung einer Hysterese (**H**), anders ausgedrückt, einer das Ausströmintervall (**IE**) verlängernden zusätzlichen Dauer, bei einem Herzschrittmacher mit einem Stimulationszähler (**M**), der ausgehend von einem programmierten Maximum dekrementweise vorgeht, und einem Erfassungszähler (**N**), der ausgehend von einem programmierten Maximum dekrementweise vorgeht, wobei die beiden Zähler programmgesteuert sind und die zusätzliche Dauer (**H**) mit einem als Prozentsatz des Ausströmintervalls definierten Programmierten Bereich (**Ma**) zwischen einem Minimalwert und einem Maximalwert regelbar ist,

**dadurch gekennzeichnet, daß**

- der Stimulationszähler (**M**) nach einer Stimulation dekrementiert wird und nach einer Erfassung auf sein Maximum zurückgesetzt wird,
- der Erfassungszähler (**N**) nach einer Erfassung dekrementiert wird, wenn das Intervall (**PP**) zwischen zwei aufeinanderfolgenden Erfassungen kleiner als das um die Hysterese erhöhte und um den Programmierten Bereich verringerte Ausströmintervall ( IE + H - Ma ) ist, und nach einer Stimulation auf sein Maximum zurückgesetzt wird, und
- die Regelung der zusätzlichen Dauer (**H**) gemäß einer der beiden folgenden Gruppen von Bedingungen erfolgt:
  wenn

  • nach einer Erfassung
  • die zusätzliche Dauer (**H**) kleiner als der um ein Inkrement verringerte Maximalwert (**max**) ist,
  • und der Stimulationszähler einen Wert (**M**) zwischen 0 und dem Programmierten Maximum anzeigt, wobei die Grenzen ausgeschlossen sind,
  dann wird die zusätzliche Dauer (**H**) um ein Inkrement erhöht,
  und wenn
  • nach einer Erfassung
  • die zusätzliche Dauer (**H**) größer als der um ein Dekrement erhöhte Minimalwert (**min**) ist,
  • und der Erfassungszähler (**N**) auf 0 ist,

  dann wird die zusätzliche Dauer (**H**) um ein Dekrement verringert.

2. Verfahren nach Patentanspruch 1,
**dadurch gekennzeichnet, daß**
das Inkrement, oder das Dekrement, vorzugsweise zwischen 10 und 50 ms beträgt.

3. Verfahren nach Patentanspruch 1,
**dadurch gekennzeichnet, daß**

wenn nach einer Erfassung das Intervall (**PP**) zwischen zwei aufeinanderfolgenden Erfassungen zwischen, einerseits dem um die Hysterese erhöhten und um den Programmierten Bereich verringerten Ausströmintervall ( IE + H - Ma ), und andererseits dem um die Hysterese erhöhten Ausströmintervall (**IE + H**) liegt, der Erfassungszähler dann auf sein programmiertes Maximum zurückgesetzt wird.

EP 0 539 258 B1

M = 4

N = 2000

< O  detection  N >

M = 0  O >

N  v

M = M - 1

< N  O < M < 4  O >

< O  IE + H - Ma < PP < IE + H  N >

v

< O  H ≥ max  N >

N = 2000

< N  N = 0  O >

v

H = H + increment

<

N = N - 1

H ≥ min

v

N  v  O  v

N = 2000

H = H - décrement

<

N = 2000

v

v

<

<